# EUROPEAN PATENT APPLICATION

(11) **EP 3 011 900 A1**
(43) Date of publication of application: **27.04.2016**
(21) Application number: 14190353.4
(22) Date of filing: 24.10.2014
(51) Int. Cl.: A61B 5/0408, A61B 5/00

(54) **Electrode module, suction system and tool for biopotential monitoring**

(71) Applicant: Quickels Systems AB, 114 19 Stockholm (SE)
(72) Inventor: Sjöberg, Krister, 113 61 STOCKHOLM (SE); Sjöberg, Göran, 196 30 STOCKHOLM (SE)
(74) Representative: Platt, Timothy Nathaniel

(57) **Abstract**

A vacuum hose ECG electrode module has a disposable vacuum electrode unit (5), which can be snapped into a re-usable electrode module housing (2). The disposable electrode unit (5) has a more rigid vacuum chamber dish (7) and a pliable skin-contacting lip ring (8) permanently fixed to the rim of said dish (7).

## Description

### BACKGROUND

The present invention relates in general to electrode modules for biopotential monitoring, and in particular electrocardiogram (ECG) monitoring of the heart's electrical activity through the skin at various points on the torso, ankles and wrists.

### RELATED ART

US 8 406 843 describes a disposable stick-on ECG monitoring electrode which does not use vacuum suction. It proposes a way to deal with problems which it describes in the fourth paragraph of column 1: "The strength and accuracy of the signals from the ECG electrodes to the monitor is dependent on motion artifacts caused by the movement of the patient's skin relative to the electrode. This movement can cause extraneous signals, shifting the desired signal baseline. Abrading the skin reduces the electrical potential and minimizes the impedance of the patient's skin, thereby reducing motions artifacts and improving the biopotential electrical signal. Typically, the patient's skin is prepared prior to applying the electrode. Preparatory abrasion removes a portion of the epidermis or external skin layer and is usually performed by rubbing the patient's skin with a rough surfaced material followed by cleaning the abraded area with alcohol. If the electrical potential of one or more electrodes is too great and the signal is not adequate, the electrode(s) must be removed, the site further abraded, and the electrodes (s) reapplied. This procedure is not only time consuming, but may be painful to the patient. The processes may also cause emotional distress to the patient as the patient views the skin abrading procedure. The proposed solution according to this prior art document US 8 406 843 is that the disposable electrode have an abrasive surface, used to sand off and prep the skin of the patient by twisting the electrode back and forth. The abrasive electrode can also be infused with gel which will spread over the abraded skin and decrease the impedance between the skin and the electrode surface. The proposed prior art solution also includes using adhesive patch to keep the electrode in place. Any procedure which requires abrasion of multiple skin locations and the use of gel and adhesive is naturally not optimal.

In addition to stick-on disposable electrodes, various suction modules are known in the art which are connected via suction hoses to a central vacuum unit and a recording and analysis unit for the electrical signals. Suction electrodes have the advantage of being easy to apply and remove and do not require abrasion of the skin or the use of adhesives.

One presently known suction cup module is described in WO 2011 /046490 having a housing which can be coupled to a hose for transmitting vacuum and electrical signals. A disposable electrode disc or cup has a central metal electrode, which has a flat skin-contacting surface and a knob for attachment to/detachment from a block in the module, and is surrounded by a plastic disc portion with a flexible skirt to create a vacuum chamber and seal against the skin of the patient.

However, none of the related art discloses or hints at how to achieve the solutions provided by the present invention.

### OBJECT OF THE INVENTION

A complex of difficult-to-reconcile interrelated problems has up to now not been successfully addressed by the prior art devices.
1. Hygiene: Medical protocols increasingly require sterilization or disposal of electrodes before an ECG unit is used for a new patient. Sterilization is time-consuming and thus expensive and there is a temptation for medical personnel to skip this step under pressure to perform many ECGs during a work session. Replacing entire sets of vacuum electrode modules for every new patient is prohibitively expensive. The disposable electrodes should be removable and safely discardable without the medical personnel ever touching any part of the used electrode. It is also hygienically problematic to replace, on suction units, HEPA *(high-efficiency particulate air)* filter which trap virus and bacteria, which should, ideally, be trapped as close to the patient as possible to avoid contamination of non-disposable parts of the suction unit.
2. ECG units without vacuum suction require the use of gels, adhesives as well as skin-prepping abrasion to make the disposable electrodes stick to the skin during measurement.
3. Vacuum suction ECG units are preferred by many cardiology professionals for ease of attachment and detachment and because no gel, adhesives or abrasion is needed. Only an electrolyte spray is used.
4. ECG "stress tests", of crucial importance in cardiology, usually involving cycling on an exercise bike or running on a treadmill, present significant problems because the hoses or leads can pull on the electrodes and cause them to become detached or give rise to "motion artifacts" in the ECG reading, i.e. measurement anomalies clouding the medical analysis.
5. Hair or uneven skin can break the vacuum holding the electrode in place. A disposable disc or cup made very pliable to always seal over hair or uneven skin will be susceptible to motion artifacts during ECG stress tests as the hoses unavoidably pull on the electrode modules.

### SUMMARY

Disturbances giving rise to "motion artifacts" in the ECG readings are explainable with reference to the Helmholtz double layer model where in an electrolyte such as skin moisture or a gel, two layers of opposite polarity are formed at the interface between the electrode surface and the electrolyte. Any tipping of this double layer, for example by uneven lateral forces on the electrode module when pulling on the hose, will cause a change in the capacitance and the chemical equilibrium of ions making up the Helmholtz double layer and distort the reading of the true electrical activity of the heart.

The entire complex of problems listed above finds its solution in the invention as defined in the appended main patent claim.

### BRIEF DESCRIPTION OF DRAWINGS

Embodiments of the invention will now be described in more detail with reference to the appended drawings, wherein:
Fig. 1 shows a vacuum electrode module according to the present invention in cross section.
Fig. 2 shows the same vacuum electrode module as in Fig. 1 adhering to an uneven area of skin.
Fig. 3 illustrates the effect of pulling on the hose attached to the vacuum electrode module.
Figs. 4(a)-(c) shows different views of the entire vacuum electrode module.
Figs. 5(a)-(c) show an exploded view and two cross sectional views of a disposable electrode unit.
Figs. 6-8 show the detacher tool and its use.

### DETAILED DESCRIPTION

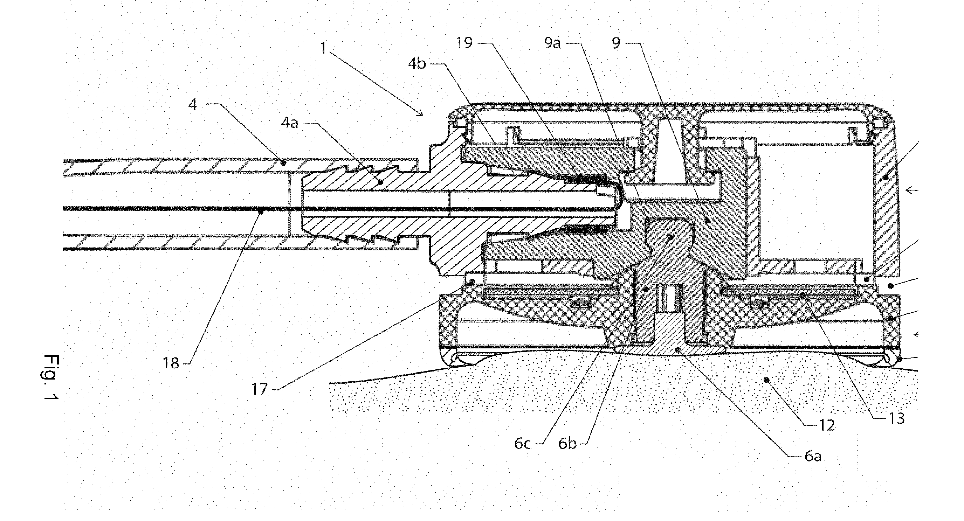

With reference to Fig. 1 a vacuum electrode module 1 is shown for use in a suction system together with an ECG monitor. Usually up to ten such vacuum electrode modules are coupled to the same suction system for ECG monitoring, each via an individual electrically conducting vacuum hose 4, which is slipped over the outer part of the nipple 4a. The nipple is part of the peripheral plastic shell 18. The inner nipple 4b is forced into the central conductive polymer block 9 in the vacuum electrode module housing 2. A disposable electrode unit 5 has a central electrode sensor element 6a-c, comprising a skin-contacting biopotential transducer sensor 6a and a snap connector 6b with a knob 6c which is snapped into a socket 9a in the electrically conducting block 9. An electrical signal conducting wire 18 within the hose 4 is connected to a contact ring 19 which is in electrical contact with the conductive polymer block 9.

When mounted, there is a peripheral groove 16 between the module housing 2 and the disposable electrode unit 5. The sealing rim 17 retains the vacuum within the electrode module housing 2 and the disposable electrode unit 5. Surrounding said electrode sensor element 6a-c is a vacuum chamber dish 7 which is made of relatively rigid plastic, in this embodiment of injection molded polypropylene thermosetting resin. Extending from the periphery of said vacuum chamber dish 7 is a circumferential lip 8 of a flexible and resilient plastic or rubber, in this embodiment of thermoplastic elastomer which has been injection molded to be permanently attached to the dish 7, which is still warm when the thermoplastic lip is molded thereto.

This construction prevents tugging on the hose 4 and/or unevenness in the skin area from causing the chemical equilibrium of the double layer interface where the electrode sensor element meets the skin surface, to be disturbed, giving rise to motion artifacts in the ECG monitoring record.

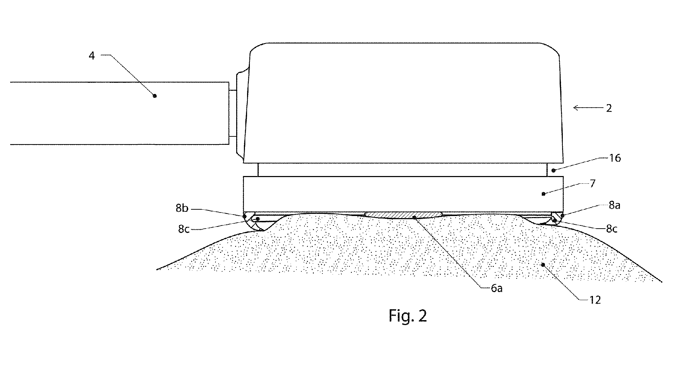

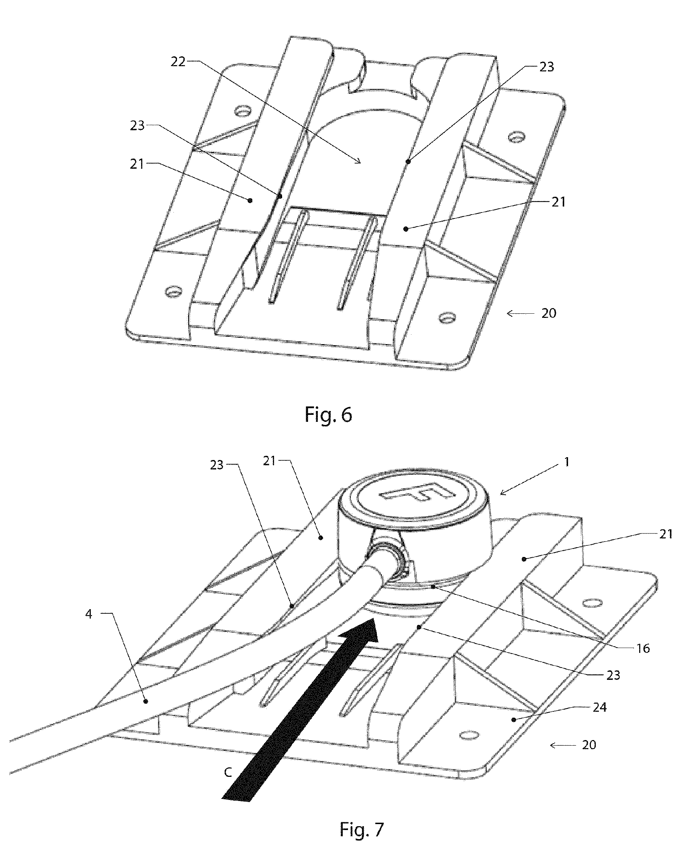

Fig. 2 shows such a situation where the skin area is uneven. The lip 8a on one side is fully compressed by the skin and the force generated by the vacuum, whereas the lip 8b on its other side is only very slightly compressed where it meets the skin, preserving the vacuum suction all around, without ever disturbing the chemical equilibrium interface where the electrode sensor element meets the skin. Also clearly visible is the peripheral groove 16 between the module housing 2 and the disposable electrode 5, the importance of which will be described below with reference to Figs. 6, 7 and 8. The lip 8 also has an interior groove 8c which facilitates folding of the lip as can be seen at 8c in Fig. 2.

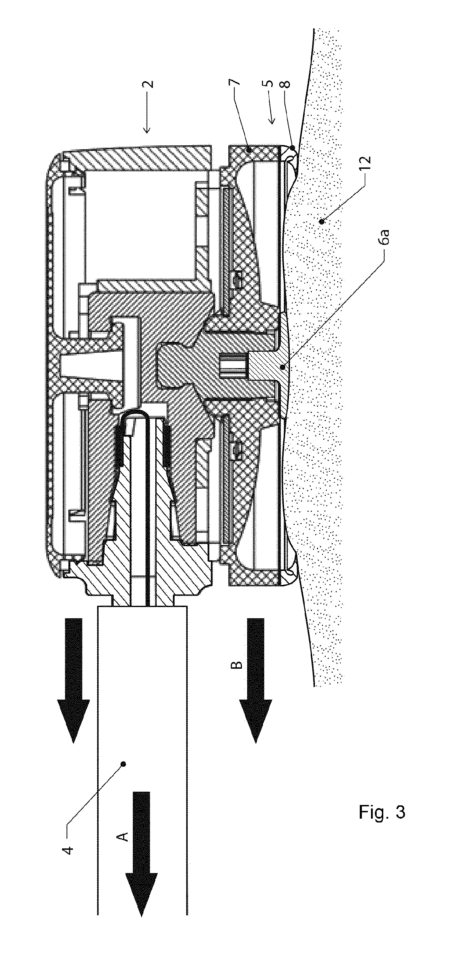

Fig. 3 shows a typical situation where, during an exercise cycle or treadmill running stress test, the vacuum hose 4 is tugged in the direction of the arrow A. By virtue of the reinforced rigidity of the vacuum chamber dish 7 together with the electrode module housing 2, the force, propagated by this pulling, at the interface between the skin-contacting electrode sensor element 6a, will be lateral in the direction of the arrow B, pulling the skin laterally, without any change in the Helmholtz double layer at said interface.

The novel construction of the disposable electrode disc unit 5 with a stabilizing more rigid portion (the vacuum chamber dish 7) and a softer, flexible and resilient lip portion 8 prevents any tipping of the biopotential transducer sensor 6a in relation to the skin 12 and at the same time ensures a complete and reliable vacuum seal around the vacuum chamber. The forces transmitted via the hose being pulled will act coplanar to the interface between the electrode surface 6a and the skin 12. If both the vacuum chamber dish 7 and the circumferential lip 8 were made in one piece of the same flexible pliable material, pulling on the vacuum hose 4 would cause the entire module to tip changing the contact between the biopotential transducer sensor 6a and the skin 12 and giving rise to motion artifacts. If, one the other hand, both the vacuum chamber dish 7 and the pliable circumferential lip 8 were made in the same more rigid material the vacuum seal risks being broken due to movement or due to hair or uneven skin, which will also distort the ECG measurements or even lead to the electrode module falling off.

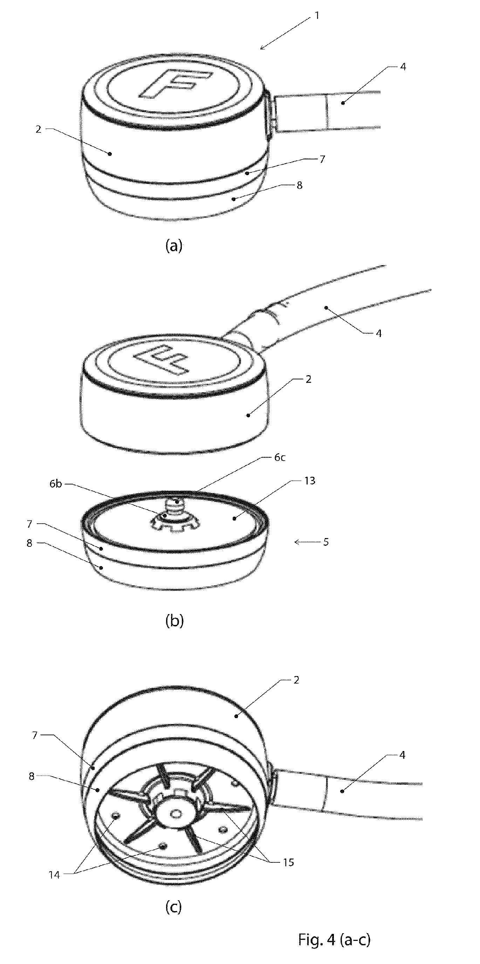

Fig. 4 (a)-(c) shows the entire coupled vacuum electrode module in three views: (a) a perspective view from above, (b) with the disposable electrode unit 2 detached from the vacuum electrode module housing 2 and (c) a view from below of the coupled module showing the vacuum chamber dish 7 with stabilizing and reinforcing ribs 15 and vacuum holes 14 to transmit suction from the hose 4. In another embodiment said ribs have been replaced by vacuum distribution channels.

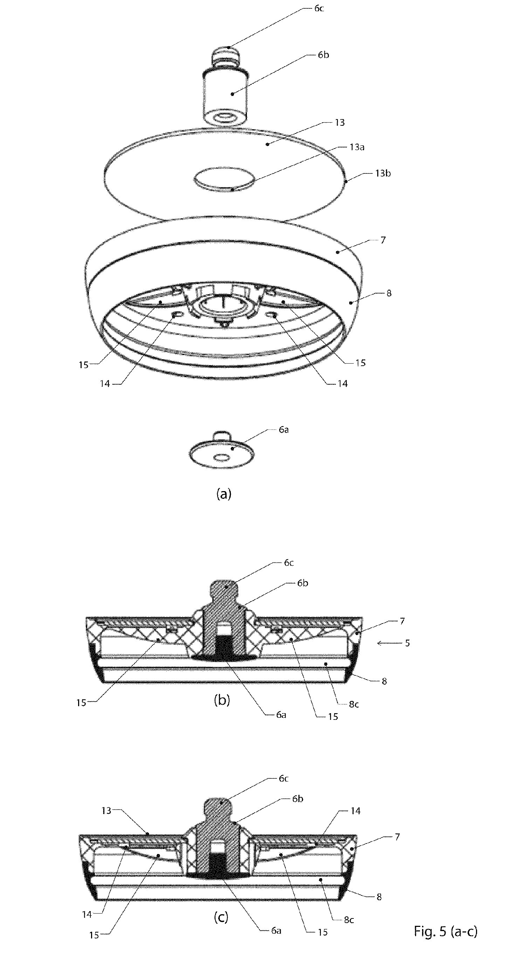

Fig. 5(a)-(c) showing the disposable electrode unit by itself in three views: (a) an exploded view showing the skin-contacting biopotential transducer sensor 6a of the electrode sensor element, the electrically conducting snap connector 6b with knob 6c. On top of the vacuum chamber dish 7 is, in this embodiment, a HEPA filter which will trap almost all particles, which can carry virus and bacteria. Fig. 5(b) shows a cross-section bisecting the reinforcing and stabilizing ribs 15. Fig. 5(c) shows a cross-section along a plane perpendicular thereto.

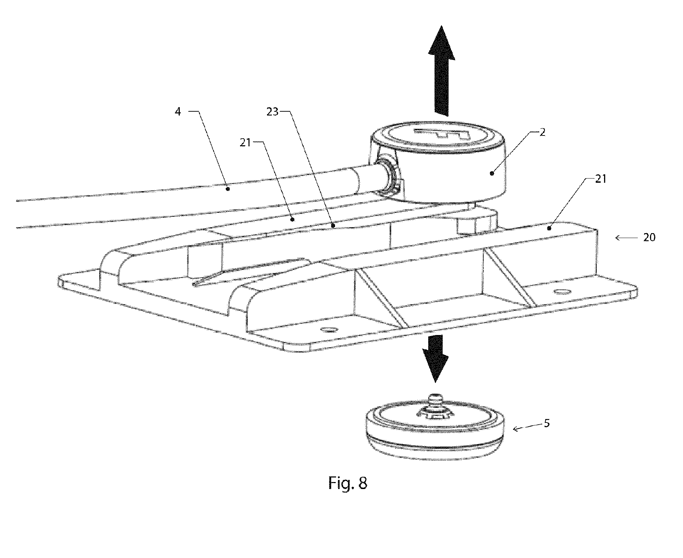

Figs. 6, 7 and 8 show the use of a specifically designed detacher tool 20 for separating the disposable electrode unit 5 from the vacuum electrode module housing 2 and disposal of the used electrode unit 5 without the medical personnel ever having to touch the used electrode unit 5. Fig. 6 shows the detacher tool 20 which has two rails 21 spaced the width of the disposable electrode unit. As can be seen in Figs. 7 and 8, the rails 21 each have a ridge 23 extending inwardly the depth of the groove 16 between the module 2 and the disposable electrode 5. To detach the used disposable electrode unit from the module housing 2, the medical worker, pushes the vacuum hose 4 and the module housing 2 between the rails in the direction of the arrow C in Fig. 7. The ridges increase in thickness gradually from the entrance in the direction of the arrow C. The ridges act as ramps to pry the disposable electrode unit 5 from the module housing 2 without having to touch it, and when it detaches it will fall through the hole 22 and into a medical waste container onto which the detacher tool is mounted via a bracket 24.

## Claims

1. Electrode module of vacuum suction type, suitable for biopotential monitoring, for example for use with an electrocardiographic monitor, comprising: a. a vacuum electrode module housing (2) with connector means (4a) suitable for receiving a hose (4) conducting vacuum and electrical signals, b. a replaceable electrode unit (5) having a central electrode sensor element (6) coaxial to a surrounding vacuum chamber dish (7), **characterized in that** from the periphery of said vacuum chamber dish (7) there extends a circumferential skin-contact lip (8) of substantially more pliable material than the material of said vacuum chamber dish (7).

2. Electrode module according to Claim 1, **characterized in that** said vacuum chamber dish (7) is made of polypropylene (PP) and said circumferential skin-contact lip (8) is made of thermoplastic elastomer (TPE).

3. Electrode module according to Claim 2, **characterized in that** said lip (8) is made of TPE having a hardness of between 30 and 60 ShoreA.

4. Electrode module according to one of the preceding claims, **characterized in that** said circumferential lip (8) is provided with an interior groove (8c).

5. Electrode module according to one of the preceding claims, **characterized in that** said replaceable electrode unit comprises a porous air filter disc 13.

6. Electrode module according to Claim 5, **characterized in that** said porous air filter disc 13 is sealed to the vacuum chamber dish (7) at its central hole (13a) and at its periphery (13b, 17).

7. Electrode module according to one of the preceding claims, **characterized in that** said module housing (2) includes a block (9) of electrically conductive polymeric material, with a nipple 4a for receiving a hose (4) and a central electrode (6a-c).

8. Suction system for use in conjunction with an apparatus for biopotential monitoring, comprising a plurality of electrode modules according to one of claims 1-7, and for each electrode module an individual hose, containing a conducting wire for biopotential signals, each hose leading to a vacuum generating unit.

9. Tool suitable for use with an electrode module as defined in one of Claims 1-7, for detaching said interchangeable electrode unit (5) from said module housing (2), **characterized in that** said tool comprises two parallel rails (21) spaced the diameter of the vacuum chamber dish (7), an entrance opening for said electrode module into the space between the two rails (21), from which two ridges (23) extend the depth of a circumferential groove (16) formed between the module housing (2) and the electrode unit (5), wherein said ridges (23) have a thickness which increases gradually from a thickness, at said entrance opening, less than the height of said circumferential groove, to a thickness greater than the height of said groove.

10. Tool according to Claim 9, **characterized in that** said tool has a mounting bracket (24) suitable for mounting said tool above a medical waste container and wherein said tool has a disposal hole (22) between said rails and disposed remote from said entrance opening for receiving a disposable electrode unit (5) as it is detached from its module housing (2).
